# EUROPEAN PATENT SPECIFICATION

(11) **EP 1 188 426 B1**
(45) Date of publication and mention of the grant of the patent: **29.10.2008**
(21) Application number: 01830582.1
(22) Date of filing: 13.09.2001
(51) Int. Cl.: A61F 13/15

(54) **A structure for realizing production lines for sanitary towels**
Struktur zur Ausführung von Produktionslinien für Monatsbinden
Structure pour la réalisation de lignes de production pour serviettes hygiéniques

(30) Priority: 19.09.2000 IT CR000004
(43) Date of publication of application: 20.03.2002
(73) Proprietor: DELTA s.r.l., 26012 Castelleone CR (IT)
(72) Inventor: Ghidelli, Luigi, 26012 Castelleone CR (IT)
(74) Representative: Mascioli, Alessandro

(56) References cited:
- WO-A-95/32696
- DE-U- 29 915 918
- GB-A- 2 075 643
- US-A- 5 492 591

## Description

The present invention concerns a structure for realizing production lines for sanitary towels, in particular for ladies, for light incontinence, baby's napkins and sanitary towels for adults.

In general, production lines for sanitary towels comprise machines and mechanic devices that manage raw materials to be treated as well as the end product.

The machines and the devices for the different working phases are at present connected by means of transmission means placed in a space below the production line, which therefore develops in horizontal rather than in vertical direction.

Furthermore, in the conventional production lines the different machines are fed through electric control panels placed besides the same.

Modular structures have also been proposed in the known art. A modular production line for disposable articles is known e.g. from EP-A-589859. Document WO 01/56523, relating to a manufacturing system for diapers and other disposable articles, is also cited as prior art under Art. 54(3) EPC.

Known structures make rather difficult any kind of servicing on the transmission means, as well as the realization of any kind of complete accident-prevention device and devices for the reduction of dust emission, as well as the possibility of noise reduction by means of sound-proof devices, and the electric system consists of a plurality of lines and connection raceways between the many operating units, which all together make the realization complicated and the managing thereof bothersome.

It is the aim of the present invention to eliminate above mentioned inconveniences.

The aim set forth is reached by means of a structure for realizing production lines for sanitary towels according to enclosed claim 1.

The 'C'-shaped frame obtained by coupling two 'L'-shaped elements, linked one to the other in correspondence of the respective ends of the core, and in a direction opposite to a horizontal plane, has the advantage of making the structure more practical and easy to transport.

For increasing the sound- proofing, the outwards turned walls of the first chamber are coated with a sound-absorbing material.

For allowing the visibility of the products along the working line, the flat means for closing are out of transparent material.

For allowing the protection of the workers and at the same time the access to the second chamber, the flat means for closing are removable.

For reducing and improving the electric connections between the power and the logic systems and the machines contained in said first chamber defined by the core of said frame, said systems are placed above the upper wing of said frame.

The elements that may be placed side by side in an indefinite number so as to form a structure of the required length.

The advantages obtained by means of the structure according to the present invention mainly consist in that the servicing of all machines and transmission systems contained in the production line is facilitated; the dust and sound emissions are reduced; a standardization and reduction of all components and spare parts is obtained; a greater safety of the production line is obtained and a greater visibility of the products during the different working phases; the splitting up of the managing logic programme of the production line is obtained, into a plurality of programmes that can be managed by the PLC s contained in the various logic systems connected to the different elements and that form the frame of the general structure, so as to facilitate the updating, the modification and the replacing of said programmes without modifying the general managing programme of the production line.

Further features and advantages of the structure according to the present invention will be described herein below relating to the enclosed drawings in which preferred embodiments are shown.
Figure 1 shows a perspective view of a portion of a structure for realizing production lines for sanitary towels according to the present invention, wherein a cross section of the 'C'-shaped frame forming the same is pointed out.
Figure 2 shows an exploded perspective view of a standard element that is connected to other similar to elements realize structures according to the present invention, wherein said 'C'-shaped frame is obtained by coupling two 'L'-shaped elements in opposite direction.
Figure 3 shows perspective view of a assembled standard element, complete with electric power and logic systems.
Figure 4 shows a perspective view of a complete structure for a production line obtained by assembling in sequence a plurality of elements like the one shown in figures 2 or 3.

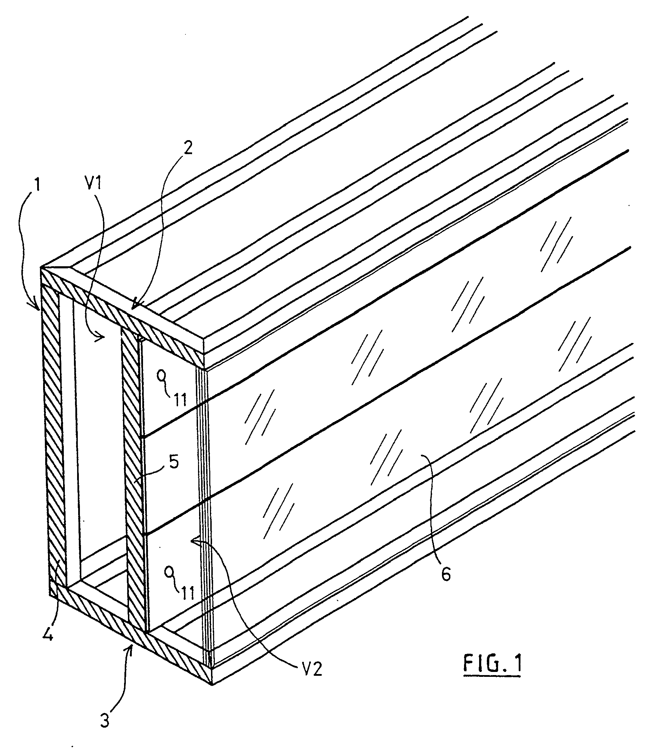

Relating to the details shown in figure 1, where a structure according to the present invention is shown, said cross structure mainly comprises a frame with a cross section having a 'C'-shape, consisting of a core 1 and two wings, an upper wing 2 and a lower wing 3.

The core 1 in turn comprises outer uprights 4 and inner uprights 5 for connection with said wings 2 and 3, spaced out so as to define a first chamber V1.

The free ends of the two wings 2 and 3 are in turn connected with flat closing means 6 so as to define a second chamber V2, comprised between said inner upright 5 and said flat closing means 6. Said chamber V1, placed behind said production line, will receive the machines, the motors and the transmission means (not shown); while said chamber V2, placed ahead of the production line, receives the raw materials and the products to be treated.

At least the walls bordering with the outside of said chamber V1 are advantageously coated with sound-absorbing material.

The closing means 6 forming the front wall of said chamber V2 are advantageously at least partially transparent.

The back wall 7 of said chamber V1 and the front wall 6 of said chamber V2 consist of removable closing means - sliding, with a shutter or a wing - so as to allow the workers an easy access to its inside.

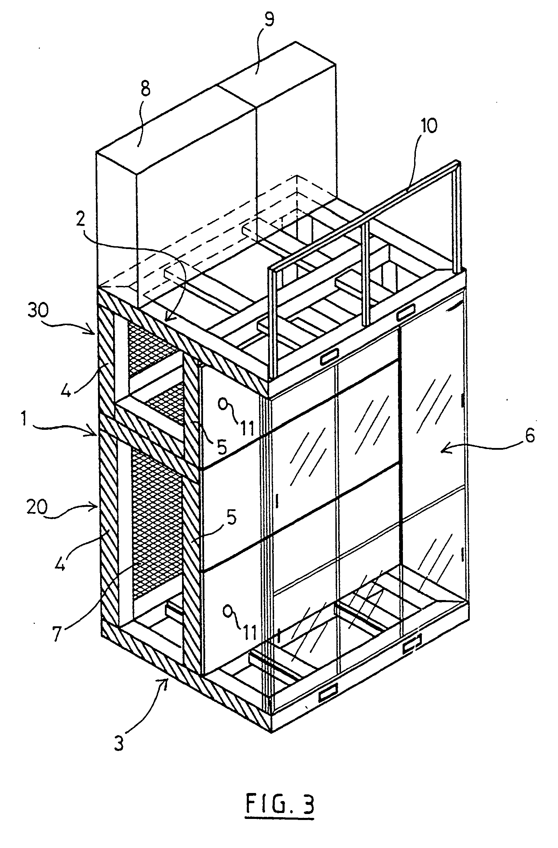

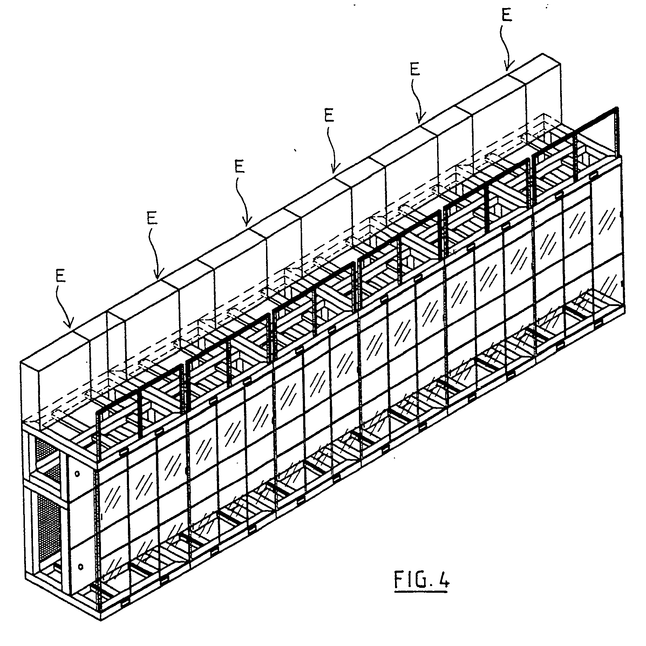

Above said upper wing 2, electric power systems 8 and logic systems 9 are applied. A safety parapet 10 is applied to the end of wing 2, as shown in figures 3 and 4, for allowing to walk safely on the upper floor of said wing 2.

The division wall between said chamber V1 and said chamber V2 has a plurality of openings 11, for allowing the passage of mechanic means and rotating shafts as well as for allowing the suction of air to be cleaned from dust.

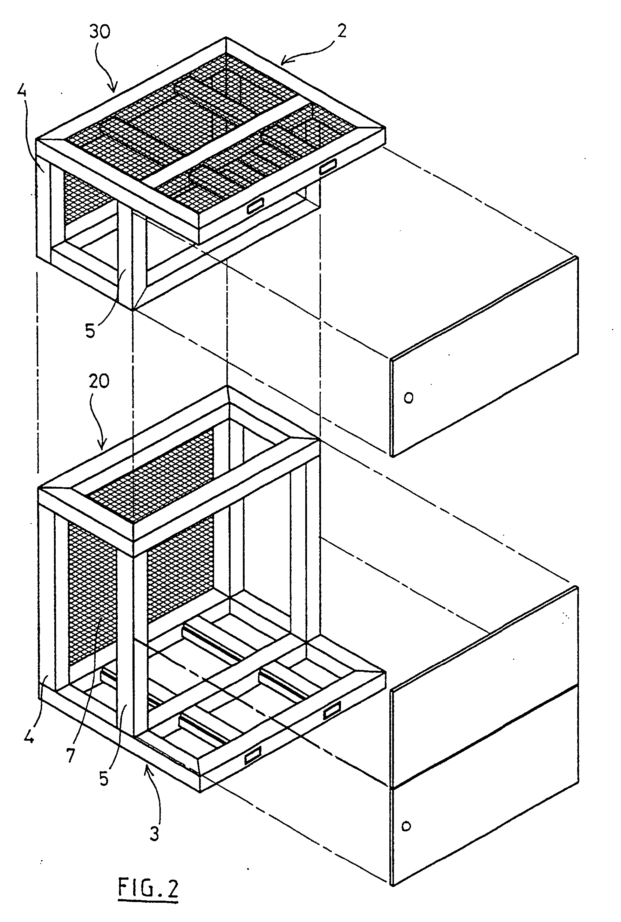

Relating now to figures 2, 3 and 4, the 'C'-shaped frame comprises two 'L'-shaped elements, a lower one 20 and an upper one 30, that may be connected one to the other in correspondence of the relating uprights 4 and 5 in a direction opposite to a horizontal plane. Said two elements 20 and 30, that may be connected one to the other, make easier the transport of said frame 1.

The frame of the production line according to the present invention is obtained by associating a plurality of elements E placed side by side and connected one to the other in a longitudinal sequence, as shown in figure 4.

## Claims

1. A structure for realizing production lines for sanitary towels, comprising a frame with a C-shaped cross section, having a hollow core (1), an upper wing (2) and a lower wing (3), outer uprights (4) and inner uprights (5), and flat closing means (6) connecting said upper wing (2) to said lower wing (3), arranged to define a first chamber (V1) between said outer uprights (4) and inner uprights (5), for containing machines, motors and transmission elements, and a second chamber (V2) between said inner uprights (5) and closing means (6), for containing raw materials and products to be treated, wherein said C-shaped frame comprises a plurality of modular elements (E) placed side by side in a longitudinal sequence, and each of said modular elements (E) is made of a lower L-shaped element (20) and an upper L-shaped element (30), linked one to the other in correspondence of their respective uprights, in a direction opposite to a horizontal plane.

2. A structure according to claim 1, **characterized in that** said first chamber (V1) has walls coated with a sound-absorbing material.

3. A structure according to claims 1 or 2, **characterized in that** said flat closing means (6) are at least partially transparent.

4. A structure according to any one of preceding claims, **characterized in that** said first chamber (V1) has a back wall (7) comprising movable shutter closing means.

5. A structure according to any one of preceding claims, **characterized in that** said flat closing means (6) of the second chamber (V2) comprise movable shutter closing means.

6. A structure according to any one of preceding claims, **characterized by** electric power (8) and logic systems (9) which are applied above said upper wing (2).

7. A structure according to any one of preceding claims, **characterized by** a division wall between said first chamber (V1) and said second chamber (V2) having a plurality of openings (11) for allowing the passage of mechanic means and rotating shafts and for allowing the suction of air to be cleaned from dust.

## Patentansprüche

1. Eine Struktur zur Ausführung von Produktionslinien für Damenbinden, bestehend aus einem Rahmen mit C-förmigem Querschnitt und Hohlkern (1), einem oberen (2) und unteren (3) Flügel, äußeren (4) und inneren (5) Stützen, sowie flache Abdeckungen (6), die den oben genannten oberen Flügel (2) mit dem unteren verbinden (3), wodurch eine erste Kammer (V1) zwischen besagten äußeren (4) und inneren (5) Stützen entsteht, die Maschinen, Motoren und Getriebeelemente umfassen kann, sowie eine zweite Kammer (V2) zwischen besagten Innenstützen (5) und Abdeckungen (6), die für Rohstoffe, bzw. Ausgangsmaterial und den zu bearbeitenden Produkten bestimmt ist. Der erwähnte C-förmige Rahmen ist aus zahlreichen modularen und aneinander gereihten Elementen (E) zusammengesetzt. Jedes dieser Elemente (E) wiederum besteht aus einem unteren L-förmigen Bestandteil (20) und einem oberen L-förmigen Bestandteil (30), die in Übereinstimmung mit ihren entsprechenden Stützen miteinander verbunden sind, und zwar entgegengesetzt einer horizontalen Fläche.

2. Eine Struktur nach Anspruch 1, **dadurch gekennzeichnet, dass** die Wände der oben erwähnten ersten Kammer (V1) mit einem schalldämpfenden Material beschichtet sind.

3. Eine Struktur nach Anspruch 1 oder 2, **dadurch gekennzeichnet, dass** die oben erwähnten flachen Abdeckungen (6) zumindest teilweise durchsichtig sind.

4. Eine Struktur nach einem der vorhergehenden Ansprüche, **dadurch gekennzeichnet, dass** besagte erste Kammer (V1) mit einer Rückwand (7) ausgestattet ist, bestehend aus verschiebbaren Schließklappen.

5. Eine Struktur nach einem der vorhergehenden Ansprüche, **dadurch gekennzeichnet, dass** die besagten flachen Abdeckungen (6) der zweiten Kammer (V2) mit verschiebbaren Schließklappen ausgestattet sind.

6. Eine Struktur nach einem der vorhergehenden Ansprüche, **gekennzeichnet durch** Stromversorgung (8) und logische Systeme (9), die an den besagten oberen Flügel (2) angebracht werden.

7. Eine Struktur nach einem der vorhergehenden Ansprüche, **gekennzeichnet durch** eine Trennwand zwischen besagter erster Kammer (V1) und zweiter Kammer (V2), die mehrere Öffnungen besitzt (11), um einen Durchgang sowohl für mechanische Vorrichtungen als auch für Drehachsen zu bilden, sowie das Absaugen der vom Staub zu reinigenden Luft zu ermöglichen.

## Revendications

1. Structure pour la réalisation de lignes de production de serviettes hygiéniques, comprenant un châssis avec une coupe en C, disposant d'un noyau creux (1), une aile supérieure (2) et une aile inférieure (3), des rebords externes (4) et des rebords internes (5), et des dispositifs de fermetures plats (6) reliant ladite aile supérieure (2) à ladite aile inférieure (3), disposés de manière à définir une première chambre (V1) entre lesdits rebords externes (4) et rebords internes (5) pour contenir les machines, les moteurs et les éléments de transmission, et une deuxième chambre (V2) entre lesdits rebords internes (5) et dispositifs de fermeture (6) pour contenir les matériaux bruts et les produits à traiter, où ledit châssis en C comprend une multitude d'éléments modulaires (E) placés côte à côte dans une séquence longitudinale et chacun desdits éléments modulaires (E) est constitué d'un élément inférieur en L (20) et d'un élément supérieur en L (30), reliés l'un à l'autre au niveau de leurs rebords respectifs, dans une direction opposée à un plan horizontal.

2. Structure selon la revendication 1, **caractérisée par le fait que** ladite première chambre (V1) possède des parois revêtues d'un matériau d'insonorisation.

3. Une structure selon les revendications 1 ou 2, **caractérisée par le fait que** lesdits dispositifs de fermeture (6) sont au moins partiellement transparents.

4. Structure selon l'une quelconque des revendications précédentes, **caractérisée par le fait que** ladite première chambre (V1) possède une paroi arrière (7) comprenant des dispositifs de fermeture à volet mobile.

5. Structure selon l'une quelconque des revendications précédentes, **caractérisée par le fait que** lesdits moyens de fermeture plats (6) de la deuxième chambre (V2) comprennent des dispositifs de fermeture à volet mobile.

6. Structure selon l'une quelconque des revendications précédentes, **caractérisée par** une alimentation électrique (8) et des systèmes logiques (9) qui sont appliqués au-dessus de l'aile supérieure (2).

7. Structure selon l'une quelconque des revendications précédentes, **caractérisée par** une cloison entre ladite première chambre (V1) et ladite deuxième chambre (V2) disposant d'une multitude d'ouvertures (11) pour permettre le passage des dispositifs mécaniques et des arbres pivotants et pour permettre l'aspiration de l'air devant être nettoyée de la poussière.
